(19) Europäisches Patentamt
European Patent Office
Office européen des brevets

(11) **EP 3 932 351 A1**

(12) **EUROPEAN PATENT APPLICATION**

(43) Date of publication:
**05.01.2022 Bulletin 2022/01**

(51) Int Cl.:
**A61B 18/14** *(2006.01)*     **A61B 90/00** *(2016.01)*
**A61B 18/00** *(2006.01)*

(21) Application number: **20183219.3**

(22) Date of filing: **30.06.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **OREN, Eitan
5656 AE Eindhoven (NL)**

• **SHLAIN, Adar
5656 AE Eindhoven (NL)**
• **COHEN, Stavit
5656 AE Eindhoven (NL)**
• **IBRAGIMOV, Zalman
5656 AE Eindhoven (NL)**
• **FAINGERSH-KLEBANOV, Anna
5656 AE Eindhoven (NL)**

(74) Representative: **Philips Intellectual Property &
Standards
High Tech Campus 52
5656 AG Eindhoven (NL)**

(54) **CONTACT SENSING FOR AN ABLATION CATHETER**

(57)     A mechanism for predicting whether or not contact occurs between an ablation therapy catheter and an anatomical structure. The mechanism proposes two modes of operation. A first mode is used if the ablation therapy catheter is currently performing ablation, e.g. current is being supplied by an ablation controller to electrodes of the ablation therapy catheter, whereas a second mode is used if the ablation therapy catheter is not currently performing ablation. The two modes use two different algorithmic processes to predict whether or not contact occurs between the ablation therapy catheter and the anatomical structure.

FIG. 2

**Description**

TECHNICAL FIELD

The present disclosure relates generally to contact sensing for an ablation catheter.

BACKGROUND

Ablation catheters are becoming increasingly used in the medical treatment of patients. For example, atrial fibrillation (AF) is an abnormal heart rhythm characterized by rapid and irregular beating of the atria, and may be associated with heart palpitations, fainting, lightheadedness, shortness of breath, or chest pain. The disease is associated with an increased risk of heart failure, dementia, and stroke. AF may be caused by electrical pulses generated by secondary pacers at the ostium of the pulmonary veins. Accordingly, one way of treating AF is by pulmonary vein isolation, which can include ablating the inner wall of the left atrium to form lesions that isolate the ostium of the pulmonary veins from the rest of the left atrium.

Ablation can be performed in various ways, including radiofrequency (RF) ablation, ultrasonic ablation, pulsed field ablation and cryoablation. RF ablation is a conventional ablation procedure that involves powering an electrode of an ablation catheter to create contiguous, transmural lesions using heat energy.

To provide effective ablation, it would be advantageous to properly position the ablation catheter against the anatomical structure or feature to be ablated. It would be particularly advantageous if a contact between the ablation catheter and the anatomical structure could be identified and, preferably, measured.

Several catheters have been developed in order to sense the contact force level. These catheters tend to rely upon complex mechanical elements (springs for example) at the tip of the ablation catheter to detect whether or not contact is made.

Other known approaches, such as that suggested by WO 2019/215721 A1, propose to measure impedance between electrodes in order to estimate a contact force applied by a catheter to tissue of a patient. Yet another approach is suggested by US 2012/283715 A1, which proposes to correlate a capacitive measurement value to determine a contact force between an ablation catheter and tissue (i.e. an anatomical structure).

SUMMARY

Aspects of the present disclosure provide devices, systems, and methods for predicting a contact between an ablation therapy catheter and an anatomical structure (e.g. an organ, blood vessel or the like).

According to an embodiment, there is provided a processor circuit for predicting if an ablation therapy catheter is in contact with an anatomical structure, the processor circuit being configured for communication with an ablation therapy catheter comprising two or more electrodes. The processor circuit is formed of an input interface and a data processor.

The input interface is configured to obtain from the two or more electrodes of the ablation catheter, at least one electrical response of each electrode of the ablation therapy catheter to one or more electric fields in the anatomical structure; and from the electrodes of the ablation therapy catheter or a controller of the ablation therapy catheter, an indicator signal responsive to an indication of whether or not the ablation therapy catheter is ablating the anatomical structure.

The data processor is communicatively coupled to the input interface, and is configured to in response to the indicator signal indicating that the ablation therapy catheter is not ablating the anatomical structure, use a first algorithmic process to process a first set of one or more electrical responses of one or more electrodes of the ablation therapy catheter to predict whether or not the ablation therapy catheter is in contact with the anatomical structure; and in response to the indicator signal indicating that the ablation therapy catheter is ablating the anatomical structure, use a second, different algorithmic process to process a second set of one or more electrical responses of one or more electrodes of the ablation therapy catheter to predict whether or not the ablation therapy catheter is in contact with the anatomical structure; and output a predictive signal responsive to the prediction of whether or not the ablation therapy catheter is in contact with the anatomical structure.

The present disclosure recognizes that, whilst a contact between an anatomical structure and an ablation catheter can be identified by monitoring the electrical response of electrodes on the ablation catheter, performing an ablation can result in the accuracy of a contact detection process to be negatively affected.

The present disclosure proposes to use a different algorithmic process to identify/predict whether (or not) the ablation catheter is in contact with the anatomical structure during ablation compared to when no ablation is performed. This enables conventional contact detection approaches (which are generally highly accurate) to be used when ablation is performed and alternative (potentially less accurate) contact detection approaches to be used when ablation is not performed.

The two or more electrodes of the ablation therapy catheter may comprise one or more electrodes used to apply an ablation treatment to the anatomical structure. The skilled person would appreciate how an electrode can be used to apply an ablation treatment through appropriate control, for example, of a current applied to an electrode (e.g. from the heat generated by applying a current to an electrode) or of a voltage at an electrode (e.g. to provide high voltage pulses for performing pulsed field ablation).

In some examples, the first algorithmic process processes the electrical responses of a first set of one or more electrodes of the ablation therapy catheter; and the second algorithmic process processes the electrical responses of a second, different set of one or more electrodes of the ablation therapy catheter.

The present disclosure also recognizes that the effect of ablation is particularly significant on a certain subset of the electrodes (which are generally one or more electrodes whose electrical responses can detect a contact with greater accuracy than other electrodes). Thus, present disclosure proposes to use different sets of one or more electrodes, depending upon whether or not ablation is being performed, to decide whether or not contact is made between the catheter and the anatomical structure.

In some examples, the number of electrodes in the second set of electrodes is greater than the number of electrodes in the first set of electrodes.

The first set of one or more electrodes may comprises a most distally positioned electrode of the ablation therapy catheter. A most distally positioned electrode can improve the accuracy of detecting whether contact with the anatomical structure is made (as it may be more sensitive than other elements, or more representative of the position of the catheter), but may be more significantly affected by an ablation process. In some examples, the second set of one or more electrodes does not comprise the most distally positioned electrode of the ablation therapy catheter.

In some examples, the first set of electrodes comprises at least one of the one or more electrodes used to apply an ablation treatment to the anatomical structure. The second set of electrodes preferably does not comprise the at least one of the one or more electrodes used to apply an ablation treatment to the anatomical structure.

It is also herein identified that the electrical response of electrodes that are used to perform ablation ("ablation electrodes") are the most significantly affected when ablation is performed, significantly affecting their accuracy and reliability in detecting a contact and/or contact force. This embodiment recognizes that it would be advantageous to rely upon the electrical response of non-ablation electrodes during ablation.

However, it is also recognized that the electrical response of non-ablation electrodes are generally less sensitive to contact with an anatomical structure. (e.g. because non-ablation electrodes are typically smaller than ablation electrodes, and are not necessarily positioned at an optimal location).

In some embodiments, where the two or more electrodes includes electrodes used to apply an ablation treatment, the second set of electrodes does not comprise any of the electrodes used to apply an ablation treatment to the anatomical structure. This enhances the effects of avoiding use of ablation electrodes during an ablation treatment.

Preferably, the first algorithmic process comprises determining an impedance measure, of an impedance between two electrodes, using the electrical responses of the two electrodes to an electric field generated at one of the two electrodes. It has been identified that an impedance measure can reliably and accurately predict whether or not the catheter is in contact with the anatomical structure.

Preferably, at least one of the two electrodes associated with the impedance measure is an ablation electrode used to apply an ablation treatment to the anatomical structure. At least one of the two electrodes associated with the impedance measure is preferably a most distally positioned electrode of the ablation therapy catheter.

In some examples, the second algorithmic process does not comprises determining an impedance measure of an impedance between two electrodes. It has been recognized that impedances measures are significantly affected by ablation, and it would therefore be preferably if impedance measurements are not used to predict whether or not there is contact during an ablation procedure.

The processor circuit may comprise an output interface configured to provide the predictive signal responsive to the prediction of whether or not the ablation therapy catheter is in contact with the anatomical structure.

The processor circuit may further comprise an electrical signal generator configured to control a current supplied to each of the two or more electrodes to thereby control an electric field generated by each electrode.

In some examples, each of the first and second algorithmic processes are configured to predict a contact pressure applied by the ablation therapy catheter, the contact pressure thereby indicating whether or not the ablation therapy catheter is in contact with the anatomical structure.

In some examples, the electrical response of each electrode of the ablation catheter is a voltage response of each electrode to electric fields generated by each other electrode of the ablation catheter.

In some examples, the two or more electrodes comprise at least a first electrode, a second electrode and a third electrode and the first algorithmic process is configured to process an electrical response of the first electrode to an electric field generated by the first electrode and the second algorithmic process is configured to process an electrical response of the second electrode to an electric field generated by the third electrode.

In some examples, the second algorithmic process is further configured to process an electrical response of the first electrode to an electric field generated by the first electrode, wherein the second algorithmic process uses the electrical response of the second electrode to an electric field generated by the third electrode to account for disturbances in the electrical response of the first electrode to an electric field generated by the first electrode resulting from the ablation being performed by the therapy catheter.

There is also proposed a method for predicting if an ablation therapy catheter, comprising two or more electrodes, is in contact with an anatomical structure.

The method comprises: obtaining at least one electrical response of each electrode of the ablation therapy catheter to one or more electric fields in the anatomical structure; obtaining from the electrodes of the ablation therapy catheter or a controller of the ablation therapy catheter, an indicator signal responsive to an indication of whether or not the ablation therapy catheter is ablating the anatomical structure, in response to the indicator signal indicating that the ablation therapy catheter is not ablating the anatomical structure, using a first algorithmic process to process a first set of one or more electrical responses of one or more electrodes of the ablation therapy catheter to predict whether or not the ablation therapy catheter is in contact with the anatomical structure; and in response to the indicator signal indicating that the ablation therapy catheter is ablating the anatomical structure, using a second, different algorithmic process to process a second set of one or more electrical responses of one or more electrodes of the ablation therapy catheter to predict whether or not the ablation therapy catheter is in contact with the anatomical structure; and outputting a predictive signal responsive to the prediction of whether or not the ablation therapy catheter is in contact with the anatomical structure.

There is also proposed a computer program product comprising code which, when executed by a processor circuit, causes the processor circuit to perform the steps of any herein descried method. There is also proposed a non-transitory computer-readable medium or data carrier comprising or carrying the computer program product.

The present disclosure also proposes a computer program (product) comprising instructions which, when the program is executed by a computer or processing system, cause the computer or processing system to carry out (the steps of) any herein described method.

The skilled person would be readily capable of adapting any herein described method to reflect embodiments of herein described apparatus, systems and/or processor circuits, and vice versa. A similar understanding would be made by the skilled person with respect to a computer program (product).

The processor circuit can be comprised in an apparatus for determining a state of contact between an ablation catheter, such as for example an RF ablation catheter, and a tissue of a subject before, during and after ablation procedure. In embodiments such apparatus may thus comprise a computer (mobile (phone, tablet laptop) or stationary (desktop or workstation or console of a medical device such as dielectric imaging or mapping system). In embodiments the processor circuit or device may be comprised in a console of a medical device such medical device may be a dielectric or electromagnetic imaging or mapping system.

Additional aspects, features, and advantages of the present disclosure will become apparent from the following detailed description.

BRIEF DESCRIPTION OF THE DRAWINGS

Illustrative embodiments of the present disclosure will be described with reference to the accompanying drawings, of which:

Fig. 1    illustrates an ablation system;
Fig. 2    is a flowchart illustrating a method;
Fig. 3    illustrates voltage responses for use in an embodiment;
Fig. 4    illustrates an ablation catheter;
Fig. 5    illustrates an ablation system;
Fig. 6    illustrates a processor circuit;
Fig. 7    is a flowchart illustrating another method.

DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates.

The present invention proposes a mechanism for predicting whether or not contact occurs between an ablation therapy catheter and an anatomical structure. The mechanism proposes two modes of operation. A first mode is used if the ablation therapy catheter is currently performing ablation, e.g. current is being supplied by an ablation controller to electrodes of the ablation therapy catheter, whereas a second mode is used if the ablation therapy catheter is not currently performing ablation. The two modes use two different algorithmic processes to predict whether or not contact occurs between the ablation therapy catheter and the anatomical structure.

Embodiments are based on the realization that an active or ongoing ablation can affect the electrical response of some electrodes of the catheter, particularly ablation electrodes. This means that (during ablation) the electrical response will become unreliable for detecting whether contact occurs. It is therefore proposed to use another process to detect contact during ablation (where that process may be less accurate that using the electrodes of the catheter which are more heavily affected by the ablation).

The underlying concept may be used to improve an ablation process performed using an ablation catheter, and may be implemented in any suitable ablation system.

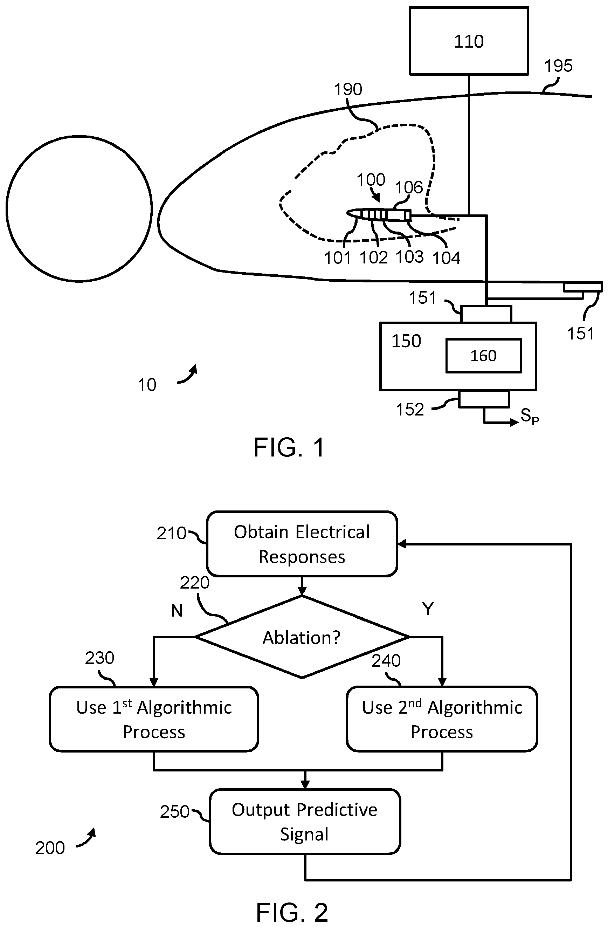

Fig. 1 provides a graphical view of an ablation system 10, comprising an ablation therapy catheter 100. The ablation therapy catheter is illustrated as being in operation within an anatomical structure 190 (e.g. a heart) of a patient/subject 195. Other examples of suitable anatomical structures will be described later.

The ablation therapy catheter 100 comprises two or more electrodes 101-104 mounted upon a flexible elongate member 106. In some embodiments, the catheter comprises 4 electrodes (as illustrated), namely: a first electrode 101, a second electrode 102, a third electrode 103 and a fourth electrode 104. However, the catheter can include other numbers of electrodes, including 2, 4, 6, 14, 16, 20, 24, 30, 60, or any other suitable number of electrodes. The flexible elongate member 106 enables the ablation therapy catheter 100 to be positioned at desired locations within the anatomical structure 190.

One or more of the electrodes 101-104 of the ablation therapy catheter 100 are designated "ablation electrodes", which can controllably ablate the anatomical structure 190. Ablation can be carried out by controlling an alternating current ("ablation current") supplied to the ablation electrode(s) 101-104 in order to appropriately control an output (e.g. electrical field intensity output by or heat of) the ablation electrode(s).

In one example of ablation, radiofrequency ablation (RFA), heat is generated by supplying a medium frequency alternating current (e.g. in the range of (in the range of 350-500 kHz) to each ablation electrode. The heat generated by the ablation electrode(s) ablates/damages surrounding tissue (i.e. tissue of the anatomical structure 190).

However, ablation electrodes may be used to perform other forms of ablation, such as pulsed field ablation (PFA), sometimes called electroporation. Pulsed field ablation is a non-thermal ablation procedure that uses high voltage bursts which are delivered in close proximity to tissue, which results in nanoscale pores (electroporation) being induced in cell membrane, which can result in cell death (i.e. ablation of tissue).

Ablation electrodes may be controlled to perform a combination of different ablation procedures during an overall ablation procedure (e.g. a combination of RFA and PFA).

Any number of the electrodes 101-104 may act as an ablation electrode, so that there may be only 1 ablation electrode or a plurality of ablation electrodes. Preferably, the ablation therapy catheter 100 is configured so that at least the most distally positioned electrode 101 (a "first electrode") can be controlled to selectively ablate nearby tissue, e.g. generate or not generate heat, i.e. is an ablation electrode.

In the context of the present disclosure, "ablation" is considered to be actively occurring (i.e. ongoing) when the ablation electrode(s) are purposively controlled to damage tissue (for an intended medical purpose).

For example, ablation occurs during RFA when sufficient current is supplied to one or more ablation electrodes that results in the ablation electrodes heating to a degree that would result in suitable ablation damage (for intended medical purposes) if brought into contact with tissue. Ablation occurs during PFA when sufficient current/voltage is supplied to the one or more ablation electrodes that results in electroporation in desired tissue (if the ablation electrode(s) were brought sufficiently proximate to the desired tissue).

The operation of the ablation electrode(s) 101-104 of the ablation therapy catheter 100 can be controlled by an ablation controller 110 of the ablation system 10. The ablation controller 110 may, for example, control the magnitude of a current supplied to the ablation electrode(s) 101-104. In particular, the ablation controller 110 may be electrically connected (e.g. by one or more wires positioned within the flexible elongate member 106) to the ablation electrode(s) 101-104, to thereby control a current supplied thereto.

In particular, the ablation controller 110 may comprise an electrical signal generator configured to control the electrodes of the catheter to emit electrical signals, including voltages, impedances, and currents.

The magnitude of the current supplied to the ablation electrode(s) 101-104 may be automatically controlled (e.g. to meet some predetermined ablation scheme) or manually controlled.

For example, the ablation controller 110 may be configured to receive a location of the catheter 100 (e.g. from a mapping system), determine if the location matches a predetermined location where ablation is to be performed, and performing ablation if the location matches the predetermined location. A suitable process for automatic ablation is described by the U.S. Patent Application having publication number No. 2018/310987, titled "Systems and Processes for Map-Guided Automatic Cardiac Ablation".

The ablation electrode(s) may be irrigated or non-irrigated. Irrigated electrodes may permit the delivery of fluid (e.g. sterile fluid such as saline) to the irrigations to cool the electrode interface. This can allow higher power delivery for a longer duration without the formation of coagulum. The catheter may be appropriately adapted to permit the delivery of water (e.g. comprise a fluid line or the like). The delivery of fluid may be controlled by the ablation controller.

Electrodes 101-104 not used for ablation may be labelled "non-ablation electrodes".

These electrodes may be used, optionally together with the ablation electrodes, to map the anatomical structure and/or determine a relative location of the catheter with respect to the anatomical structure. This process is, however, entirely optional.

Approaches for using electrodes to map anatomical structure (body volumes) and visualize the locations of catheters within the map can be found in, for example, U.S. Patent No. 10,278,616, titled "Systems and Methods for Tracking an Intrabody Catheter," filed May 12, 2015, and U.S. Patent No. 5,983,126, titled "Catheter Location System and Method," filed August 1, 1997, the entireties of which are hereby incorporated by reference. Thus, the electrodes may be in electrical communication with a mapping system (not shown) for mapping the anatomical structure and/or identifying a relative location of the catheter with respect to the anatomical structure.

In the illustrated example, the first electrode 101 is an ablation electrode. The second 102, third 103 and fourth 104 electrodes are non-ablation electrodes. However, other configurations will be apparent to the skilled person (e.g. using different total numbers of electrodes, ablation electrodes and/or non-ablation electrodes).

The skilled person will appreciate that appropriate placement of the ablation therapy catheter 100, and appropriate control of the magnitude of the current provided to the ablation electrode(s) thereof, facilitate ablation of (elements of features of) the anatomical structure on/in which the ablation therapy catheter operates.

Further details regarding suitable catheters and assemblies can be found in, for example, U.S. Patent No. 6,002,955, titled "Stabilized Electrophysiology Catheter and Method for Use," the entirety of which is hereby incorporated by reference, or the previously mentioned U.S. Patent Application having a publication number of No. 2018/310987.

The present disclosure relates to a mechanism for predicting whether (or not) the ablation therapy catheter 100 is in contact with the (walls or bounds of the) anatomical structure 190. In particular examples, a contact force between the ablation therapy catheter 100 and the anatomical structure 190 can be determined.

The approaches described in the present application therefore facilitate enhanced understanding of the interaction between the ablation therapy catheter and the anatomical structure, enabling a clinician to more accurately and/or precisely perform an ablation procedure. In particular, knowledge of a contact and/or contact force can be useful for efficient treatment of the organ.

Fig. 1 further illustrates a processor circuit 150 which is configured to predict whether or not the ablation therapy catheter is in contact with (tissue of) the anatomical structure 195.

In particular, the processor circuit may be configured to generate a predictive signal $S_p$, which can be output by an output interface 152, that is responsive to the prediction of whether or not the ablation therapy catheter is in contact with the anatomical structure

The processor circuit 150 comprises an input interface 151, which is configured to obtain, from two or more electrodes 101-104 "contact detection electrodes", at least one electrical response of each electrode of the ablation therapy catheter to an electric field or electric fields in the anatomical structure. This may be performed by electrically connecting the processor circuit 150 to the contact detection electrodes, e.g. via wires disposed in the flexible elongate member), to facilitate detection of electric fields. Some or all of these wires may be shared with the ablation controller 110.

Accordingly, the processor circuit 150 may comprise or an electrical signal measurer configured to detect electrical signals at the (contact detection) electrodes of the catheter, i.e. to determine an electrical response of electrodes of the catheter.

Although illustrated as a separate element, the processor circuit 150 may, in some examples, be incorporated into the ablation controller 110. In other examples, the processor circuit 150 may be incorporated into some other component of the system 10.

An electrical response of an electrode may be a measurement of a particular electrical parameter (e.g. voltage or capacitance) associated with that electrode to an electric field present in the anatomical structure. In particular, an electrical response may be a measurement of a response of a particular electrical parameter (e.g. voltage or capacitance) of that electrode to an electric field generated by an electrode of the catheter.

Different electric fields may vary in magnitude at different (near-unique) frequencies (i.e. have different frequencies). This enables a processor circuit 150 to distinguish between the effects of different electric fields upon an electrode (i.e. as different fields can be identified by virtue of the near-unique frequency), using appropriate filters to attribute different frequencies of an overall response to a different electric field.

In other words, the response of an electrode to a particular electric field can be individually identified using frequency-based filtering (as different electric fields may be associated with different sources). The skilled person would be readily capable of using appropriate digital/analog filters to attribute different frequency components of an overall response of an electrode to a particular electric field (associated with a particular frequency or frequencies).

These electric fields may be electric fields generated by the electrodes(s) 101-104 themselves, which may be in the region of 20-100 kHz. Each electrode may generate an electric field of a different frequency, so that the electrical response of an electrode to a field generated by a particular electrode can be identified. The electric fields may, for example, be controllably generated by the processor circuit and/or the ablation circuit and/or some other controller in electrical communication with the electrodes (e.g. a controller for a mapping system for identifying a relative location of the catheter).

In the context of the present disclosure, an electrical response may be any suitable electrical measurement of an electrical characteristic that is responsive to a change in a magnitude of an electric field in the vicinity of the electrode. In particular, the electrical response may include any suitable electrical measurement that responds to a change in a dielectric property (e.g. dielectric constant) in material between the contact detection electrode and the source of an electric field (to which the contact detection electrode responds). Thus, an electrical response may include a voltage response, a capacitance response and so on.

As a first example, a voltage response may be a voltage detected by an electrode at a particular frequencies (e.g. with different frequency being associated with different electric fields, e.g. different electrodes).

Thus, an overall voltage response (of an electrode) may be formed of one or more different voltage values, e.g. $(V_1, V_2, V_3)$ each associated with a different electric field, e.g. $(E_1, E_2, E_3)$. Each voltage value represents a different voltage response.

A voltage value may be a (average) voltage difference between the relevant electrode and a reference electrode or a ground/earth (at the particular frequency for the particular electric field). Each voltage value may be an average (e.g. RMS) voltage detected at an electrode for a different electric field (e.g. by filtering based on predetermined frequencies).

In the context of the present disclosure, a voltage response of an i-th electrode to an electric field generated by a j-th electrode may refer to a (average) voltage difference between the i-th electrode and a ground/reference (e.g. a voltage at a reference electrode) at a frequency of a current supplied to the j-th electrode.

As a second example, an overall capacitance response (of an electrode) may indicate a capacitance between a particular electrode and a known originating source of the electric field. Thus, an overall capacitance response may comprise two or more capacitance values, e.g. $(C_1, C_2, C_3)$ each associated with a different electric field, e.g. $(E_1, E_2, E_3)$. Each capacitance value represents a different capacitance response.

In some embodiments, the processor circuit 150 (or other component such as a mapping system) may be configured to define or control an electric field emitted by an electrode. For example, the processor circuit (or other component) may control a current supplied to an electrode. If the electrode is an ablation electrode, this current may be superimposed over the current supplied for ablation. The processor circuit 150 may, in some examples, therefore comprise an electrical signal generator configured to control the electrodes of the catheter to emit electrical signals.

Each electrode may be controlled (by the processor circuit 150 or a separate electric field controller) to emit an electric field of a different frequency. This can be performed by supplying an alternating current of the relevant frequency to each electrode (e.g. via wires disposed in the flexible elongate member 106). The frequencies emitted by the electrodes on the catheter are preferably in the region of 20-100 kHz (to avoid unintentional ablation of the anatomical structure, whilst being suited for contact (force) detection).

This approach results in a processor circuit 150 being able to identify, in a response (e.g. voltage response) of an electrode, the effect of different electric fields generated by the different electrodes. Thus, the processor circuit can determine the (electrical) response of an electrode to an electric field generated by another electrode.

As previously explained, the processor circuit 150 is configured to process one or more electrical responses of one or more contact detection electrodes to determine whether or not the catheter 100 makes contact with (tissue of) the anatomical structure. This information can be used to generate the predictive signal $S_P$ responsive to the prediction of whether or not the ablation therapy catheter is in contact with the anatomical structure.

In particular, processor circuity 160 of the processor circuit 150 may process the electrical response(s) obtained by the input interface 151, to predict whether or not there is contact between the catheter 100 and the anatomical structure 190.

This prediction may be binary (e.g. a prediction of whether or not the catheter is in contact with the anatomical structure), categorical (e.g. a prediction of a category of touch, such as "No Touch", "Touch" and "High Touch" or "Pressure Applied") and/or continuous (e.g. a touch value/measurement, whose magnitude depends upon a predicted intensity/force of a touch).

Optionally, this may be performed by predicting a contact force between the catheter and the anatomical structure (which indicates whether or not contact is made). Methods of predicting a contact force will be known to the skilled person, for example, WO 2019/215721 A1 discloses a method of predicting a contact force from impedance measurements.

Generally, without wishing to be bound by theory, an electric field amplitude increases when the contact force increases. Thus, monitoring an electrical response that is responsive to a change in electric field amplitude facilitates monitoring of a change in contact force (indicating a contact has occurred or to predict a magnitude of a contact force). The skilled person would be readily capable of correlating an electric response of an electrode to a change in contact force based on this teaching.

Fig. 1 further illustrates a reference electrode, which may monitor/provide a reference voltage level (e.g. for determining a voltage response of a particular electrode). The reference electrode may an external electrode that provides a reference voltage level. The reference electrode may, for example, be positioned on a leg of the subject (e.g. a "right leg patch"). Other suitable positions would be apparent to the skilled person, e.g. the lower back or the pelvis.

Fig. 2 is a flow-chart illustrating a process 200 performed by the processor circuit according to an embodiment.

The process comprises 200 a step 210 comprises obtaining from two or more electrodes of the ablation catheter, at least one electrical response of each electrode of the ablation therapy catheter to one or more electric fields in the anatomical structure. It is not essential that the electrical responses from all electrodes of the catheter are obtained by the processor circuit. Various electrical responses have been previously described.

Step 210 may, in some examples, comprise performing pre-filtering on obtained electrical responses. This pre-filtering can be used to account for the effect of cardiac motion, muscle contractions and/or respiratory motion.

In some examples, this pre-filtering may comprise estimating the effect of respiration (e.g. from a respiration signal responsive to respiration of the patient) or cardiac activity (e.g. from a cardiac signal responsive to a heart movement of the patient). A compensation algorithm may then be used to reduce the effect of respiration and/or cardiac activity on the obtained electrical response(s). This approach may employ a gating algorithm and/or a frequency-based filter. The frequency based filter may be used to filter cardiac activity (as this is usually of a regular frequency). The gating algorithm may be used to account for respiration (by gating to avoid respiratory movement).

Use of pre-filtering increases the reliability and accuracy of detecting a contact between the catheter and the anatomical structure.

The processor circuit is configured to obtain (e.g. via the input interface) in a step 220, from the electrodes of the ablation therapy catheter or a controller of the ablation therapy catheter, an indicator signal responsive to an indication of whether or not the ablation therapy catheter is ablating the anatomical structure.

Thus, step 220 effectively comprises determining whether ablation is being performed by the ablation therapy catheter 100, i.e. whether current for performing ablation using the ablation electrodes of the ablation therapy catheter is being supplied to the ablation electrode(s).

This step 220 can be performed by directly monitoring the ablation electrode(s), to determine whether current for ablation is being provided thereto, or by communicating with an ablation controller (which controls this current). Other approaches would be apparent to the skilled person.

The processor circuit is configured to, in response to the indicator signal indicating that the ablation therapy catheter is not ablating the anatomical structure, perform a step 230. If the ablation therapy catheter is not ablating the anatomical structure, the processor circuit instead performs step 240.

Step 230 comprises using a first algorithmic process to process a first set of one or more electrical responses of one or more electrodes of the ablation therapy catheter to predict whether or not the ablation therapy catheter is in contact with the anatomical structure.

Step 240 comprises use a second, different algorithmic process to process a second set of one or more electrical responses of one or more electrodes of the ablation therapy catheter to predict whether or not the ablation therapy catheter is in contact with the anatomical structure.

Thus, the algorithmic process used to predict whether or not the catheter is in contact with the anatomical structure changes depending upon whether or not the catheter is performing an ablation.

In a step 250, the processor circuit generates a predictive signal $S_P$ responsive to the prediction (in step 230 or 240) of whether or not the ablation therapy catheter is in contact with the anatomical structure.

The processor circuit may be configured to iteratively check whether the ablation is being performed (i.e. revert to step 220), this may be performed each time an iteration of steps 230 or 240 are performed - or may be performed after a predetermined number of iterations of step 230 or 240 are performed.

Steps 230-250 may be carried out by the data processor. The predictive signal $S_P$ may be output at an output interface.

From the foregoing, it will be apparent that the processor circuit is effectively operable in two different modes. A first mode is used if the ablation therapy catheter is currently performing ablation (e.g. current is being supplied by the ablation controller 110 to the electrodes 101-104 of the ablation therapy catheter), whereas a second mode is used if the ablation therapy catheter is not currently performing ablation.

The present disclosure recognizes that there are various options for the first and second algorithmic processes, and the invention is not intended to be limited to any particular one of these options.

The first and/or second algorithmic process may use a measurement obtained directly from an electrical response, e.g. a voltage value/response (which may be filtered to reduce noise). In another example, the first and/or second algorithmic processes may further process one or more electrical responses (e.g. of one or more electrodes) in order to generate a measurement. One example of a suitable measurement is an impedance measurement of an impedance between two electrodes (e.g. derived from voltage values for different electrodes).

The measurement may then be monitored and/or processed to predict whether or not the catheter is in contact with the anatomical structure. In particular, a contact indicator may be generated using the measurement, the contact indicator being responsive to whether or not touch is made between the catheter and the anatomical structure.

For example, the measurement may be compared to one or more thresholds magnitudes to determine whether or not there is contact between the catheter and the anatomical structure (and optionally, a level of contact). These threshold magnitudes may be preset or derived from calibration measurements, e.g. values of the measurements obtained when there is no contact between the catheter and the anatomical structure.

Different thresholds may represent different levels of contact or touch, e.g. no touch, a light touch, a heavy touch, a very heavy touch and so on. As a simple example, a single threshold may distinguish between no touch/contact (between catheter and anatomical structure) and a touch/contact between the catheter and the anatomical structure.

As another example, the measurement may be further processed to determine a contact measurement (e.g. a value indicative of a magnitude of a contact, such as a pressure applied by the catheter to the anatomical structure or a measure of movement of the anatomical structure caused by the contact).

The output of an algorithmic process may be binary (e.g. a prediction of whether or not the catheter is in contact with the anatomical structure), categorical (e.g. a prediction of a category of touch, such as "No Touch", "Touch" and "High Touch") and/or continuous (e.g. a touch value/measurement, whose magnitude depends upon a predicted intensity/force of a touch).

The first algorithmic process may use a different set of electrical (sub-) responses to the second algorithmic process in order to predict whether or not the catheter is in contact with the anatomical structure. Thus, the first and second sets of responses may be different.

In particular examples, the first algorithmic process may use electrical responses from ablation electrodes in order to predict whether or not the catheter is in contact with the anatomical structure. The electrical response of an ablation electrode tends to have a higher resolution (i.e. be more accurate) in detecting whether or not contact has been made (and is more appropriate to detecting contact, as it is an electrode that is used during the ablation process).

In some examples, the second algorithmic process may (preferably, exclusively) use electrical responses from non-ablation electrodes.

In a first scenario, the second algorithmic process only uses one or more electrical responses from non-ablation electrodes (i.e. it does not use electrical responses of ablation electrodes). This reduces the likelihood that the ablation will affect the electrical response (and/or a magnitude of this affect), and thereby increase the reliability of predicting whether or not contact is made with the anatomical structure.

In a second scenario, the second algorithmic process uses electrical response(s) of non-ablation electrodes to account for disturbances in the electrical response of the first electrode to an electric field generated by the first electrode resulting from the ablation being performed by the therapy catheter

This approach recognizes that ablation affects an accuracy of measurements obtained from ablation electrodes.

Without wishing to be bound by theory, it is believed that this is at least partly due to the heating associated with the ablation (the intended effect of RFA and a side effect of other ablation procedures) and the current applied to an electrode (to perform the ablation) causing electrode disturbances on the ablation electrode(s).

As another example, if the ablation electrode(s) are irrigated, then (during ablation) the irrigation rate can automatically increase to ensure cooling of the electrode(s) if performed appropriately. The change in the irrigation rate further introduces noise to measurements of the ablation electrode(s).

Thus, an accuracy of detecting (a level of) contact will be affected if only data from ablation electrodes is used when ablation occurs.

Preferably, the first algorithmic process comprises determining an impedance measure of an impedance between two electrodes. Preferably, the second algorithmic process does not comprise determining an impedance measure of an impedance between two electrodes.

In some examples, the first and/or second algorithmic process may operate by detecting a change (e.g. percentage) in a measurement of an electrical response (or measurement derived from one or more electrical response(s)). For example, if a change of more than a predetermined percentage of a measurement is detected, then it can be predicted that the catheter has touched tissue of the anatomical structure. As another example, if a measurement changes by more than a predetermined percentage from a calibrated or reference measurement, then it can be predicted that the catheter has touched tissue of the anatomical structure.

Thus, the first and/or second algorithmic process make take a calibration/reference measurement (e.g. previously obtained in response to a user input) of a first/second set of one or more electrical responses (or parameters derived from one or more electrical responses), and monitor a change (e.g. a percentage change) to determine whether or not contact is made.

Calibration should be repeated, to take a new calibration measurement) each time the catheter moves to a new part of the anatomical structure, e.g. the catheter is moved from one ventricle to another ventricle. This approach improves the accuracy of predicting contact.

Purely by way of example, an electrical response used by an algorithmic process may comprise a measurement of the average voltage induced by an electric field having a predetermined frequency. A calibration or reference value for this average voltage may be made (e.g. when it is known the catheter is not in contact with the tissue). The algorithmic process may predict that the catheter comes into contact with the tissue when the measurement of the average voltage changes by more than a predetermined percentage (e.g. by more than 10% or more than 5%). The algorithmic process may predict that the catheter is applying more than a minimum amount of pressure to the anatomical structure when the average voltage changes by more than a second predetermined percentage with respect to the calibration/reference measurement. In this way, the algorithmic process can detect contact, and optionally a magnitude of contact, with the anatomical structure.

Although this described example uses only a single measurement derived from a voltage response, the skilled person would appreciate how different numbers of measurements derived from different numbers of electrical responses could combined or algorithmically processed to predict the occurrence of contact or a measurement of contact force.

By way of example, the voltage response of two different electrodes could be processed to derive an impedance response or an impedance measurement between the two different electrodes. The impedance measurement could be further processed (by an algorithm) to predict whether to not the ablation therapy catheter is in contact with the anatomical structure, e.g. by monitoring percentage changes in the measurement or monitoring a threshold.

As another example, the voltage response of one electrode could be used to calibrate or error-correct the voltage response of another electrode.

In some examples, an algorithmic process may monitor a plurality of different measurements and select the most sensitive measurement for use in the algorithmic process, e.g. by detecting the largest difference between the measurement at no-touch vs. a measurement at a touch level. This process may take place during a calibration procedure.

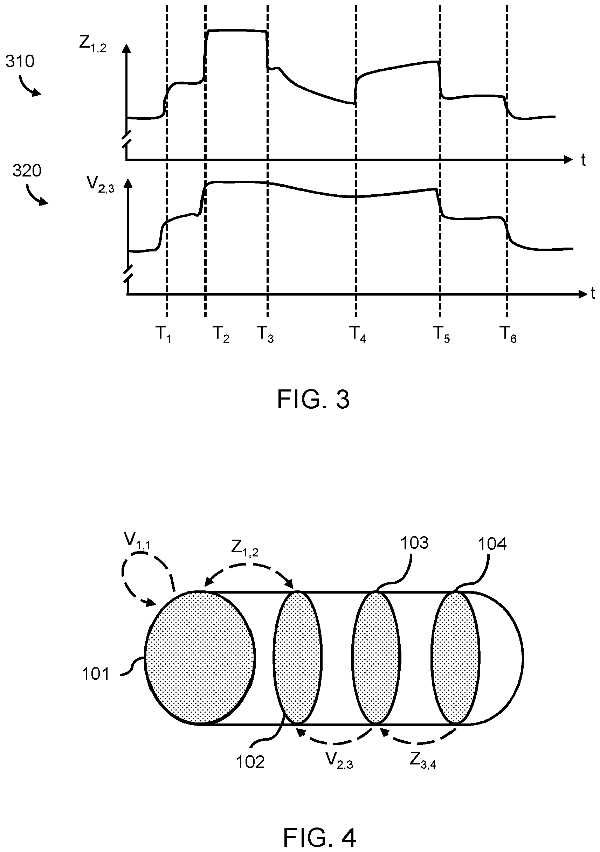

Fig. 3 is used to illustrate a working example of an embodiment of the invention. In particular, Fig. 3 illustrates a first measurement 310 and a second measurement 320 across different points in time.

In the illustrated scenario, the first measurement 310 is an impedance measurement between two electrodes, and in particular, between an ablation electrode and a non-ablation electrode. The first measurement is derived from a voltage response of each electrode.

The second measurement 320 is a measurement of a voltage response of a first non-ablation electrode to an electric field generated by a second non-ablation electrode. Thus, the second measurement is a measure of the (average) voltage induced in the first non-ablation electrode due to an electric field generated by the second non-ablation electrode.

The first algorithmic process is configured to use the first measurement 310 to predict a contact and/or contact force. The second algorithmic process is configured to use the second measurement 320 to predict a contact and/or contact force.

Thus, in the illustrated example, each algorithmic process uses a single measurement (obtained from or derived from one or more electrical response(s)) to predict a contact and/or contact force. The skilled person would appreciate that other examples may use more than one measurement (e.g. compare two measurements) to predict a contact and/or contact force.

As previously explained, the first measurement 310 is a measure of an impedance between two electrodes (e.g. a first electrode 101 and a second electrode 102). An impedance measurement can be derived from a voltage response of each electrode to an electric field generated by one of the electrodes.

For example, the impedance measurement $Z_{1,2}$ (being the impedance between a first electrode "1" and a second electrode "2" can be calculated using the following equation:

$$Z_{1,2} = A_{1,2}(V_{1,1} - V_{2,1}) + B_{1,2}V_{1,1} + C_{1,2} \qquad (1)$$

where $A_{1,2}$, $B_{1,2}$, $C_{1,2}$ are constant values, which can calculated per system during its factory calibration process (or a later calibration process), Vi,j represents the voltage response of an electrode i to an electric field generated by an electrode j (e.g. a (average) voltage difference between the electrode i and a reference electrode at the frequency of the electric field generated by the electrode j).

The approach disclosed in WO 2019/215721 A1 could be used as another example.

The second measurement 320 is a voltage response of the second electrode 102 to an electric field generated by applying a fixed alternating current to the third electrode 103. In particular, the second voltage response is an average voltage $V_{2,3}$ within a frequency band centered on the frequency of the fixed alternating current supplied to the third electrode (for the purposes of contact detection).

It has been identified that a voltage response is, by itself, less sensitive to the effects of ablation than an impedance measurement. However, an impedance measurement is (when no ablation is applied) more accurate and/or sensitive than a voltage response alone. This effect is attributed to the impedance change that occurs in the gradually changing tissue that is being ablated.

A first point in time $T_1$ indicates a transition from no-contact to contact between the catheter and the anatomical structure. A second point in time $T_2$ indicates a transition from contact to "high contact". "High contact" may indicate a state in which pressure is applied by the catheter to the anatomical structure, rather than simply contacting the anatomical structure. A third point in time $T_3$ indicates a beginning of ablation, with a fourth point in time $T_4$ indicating an end of ablation. A fifth point in time $T_5$ indicates a (de-)transition from high contact to contact between the catheter and the anatomical structure. A sixth point in time $T_6$ indicates a (de-)transition from contact to no-contact between the catheter and the anatomical structure.

As illustrated in Fig. 3, at the first point in time $T_1$ and the second point in time $T_2$ there is a marked or step change in the first 310 and second 320 measurements (indicating that these measurement are suitable for detecting contact between the catheter and the anatomical structure). As the catheter pressure against the anatomical structure, so the measurements increase.

However, between the third $T_3$ and fourth $T_4$ points in time (i.e. during ablation), the value of the first measurement 310 become less reliable (i.e. is affected by the ablation). The present invention recognizes that it would be disadvantageous to rely upon the first measurement during this time.

Thus, the present disclosure proposes to use a second algorithmic process to predict whether the catheter remains in contact with the anatomical structure. The second algorithmic process here derives or uses a measurement 320 that is less affected by the ablation (e.g. as it is a voltage response at a non-ablation electrode).

However, it is also recognized that the second measurement may be of a lower resolution/precision than the first measurement, and it would therefore be less preferable to continually rely upon this measurement if the first measurement is reliable (e.g. if no ablation is taking place).

Thus, the present disclosure provides a good compromise between reliability and resolution, and acts to ensure that there is reliable detection on contact whilst maximizing contact detection accuracy.

It is recognized that all electrical responses may be at least partially effected by an ablation procedure, resulting in drift of an electrical response of an electrode during ablation. This is illustrated in Fig. 3, as the second measurement slowly drifts during the ablation procedure. In particular, measurement can gradually decrease at a rate that increases with ablation power.

However, it is recognized that this change is less prominent when using a voltage measure, compared to an impedance measure. Thus (assuming the catheter remains in substantially the same location, e.g. is stable), the expected slow drift in a voltage response can be corrected using a linear or other pre-defined function.

Thus, in some examples, the second algorithmic process comprises using a linear and/or other pre-defined function to correct an expected drift in a measurement used to predict whether or not contact is made between the catheter and the anatomical structure (such as the impedance measurement).

Fig. 4 illustrates some electrical responses and measurements derivable therefrom that could be used for different algorithmic processes for the present disclosure.

A first measurement $Z_{1,2}$, being an impedance measurement, between the first electrode 101 and second electrode 102 is illustrated. The impedance can be calculated using a voltage response of each electrode, and in particular, a voltage value of each electrode associated with an electric field generated by one of the electrodes, as previously described (e.g. with reference to equation 1).

A second measurement, being a voltage measurement $V_{2,3}$, is the voltage response of the second electrode 102 to a fixed-frequency alternating current supplied to the third electrode 103, i.e. an electric field generated by the third electrode 103.

In the previously described examples, the first measurement $Z_{1,2}$ is used during the first algorithmic process, and the second measurement $V_{2,3}$ is used during the second algorithmic process.

However, other measurements (or combinations of measurements) could also be used, and could include a voltage induced in one electrode by another electrode, an impedance between two electrodes, a capacitance between two electrodes and so on. In particular, any suitable measurement responsive to changes in a dielectric property could be used.

One example of a suitable measurement is a voltage response $V_{1,1}$ of the first electrode 101 to a fixed-frequency alternating current supplied to/by the first electrode 101, i.e. an electric field generated by the first electrode 101. Another example is an impedance measurement of an impedance $Z_{3,4}$ between the third electrode 103 and the fourth electrode 104.

In some embodiments, more than two measurements may be used, with one or more measurements being used to correct an error in one or more other measurements.

An algorithmic process may use any combination of the possible electrical responses in order to predict whether or not contact is made between the catheter and the anatomical structure (and optionally a magnitude of contact).

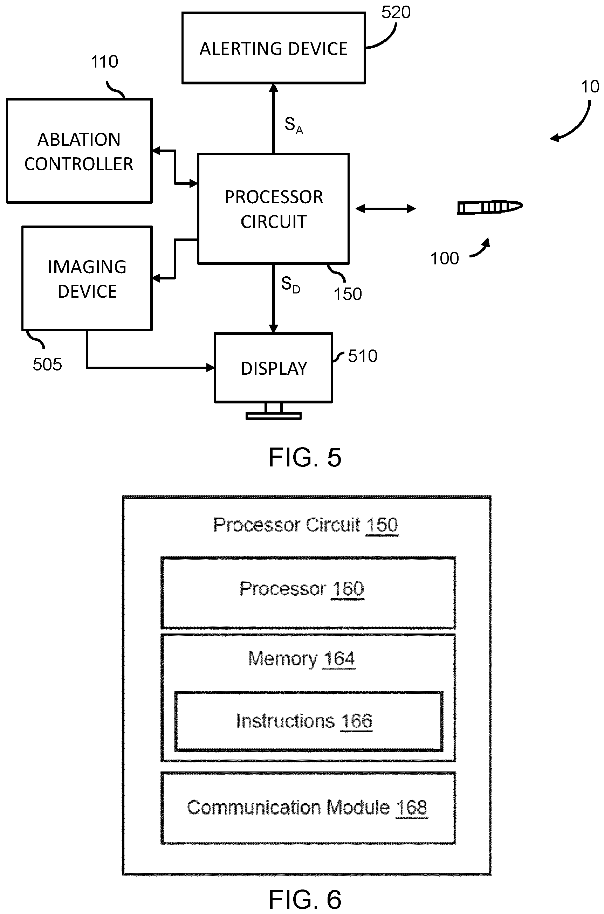

Fig. 5 illustrates some further optional elements for the ablation system 10.

The ablation system may comprise an imaging system 505 configured to generate an image of the anatomical structure. The imaging system may operate, for example, by monitoring the position of the catheter with respect to a three-dimensional space and generating an anatomical model of the anatomical structure based on the positions of the catheter . Approaches for generating an anatomical model in this manner will be apparent to the skilled person, e.g. using previously identified documents U.S. Patent No. 10,278,616 and U.S. Patent No. 5,983,126 or from Romanov, Alexander, et al. "High-resolution, real-time, and nonfluoroscopic 3-dimensional cardiac imaging and catheter navigation in humans using a novel dielectric-based system." Heart rhythm 16.12 (2019): 1883-1889.

The imaging system may control a display to generate a visual representation of the anatomical model, and optionally the position of the catheter with respect to the anatomical model.

The prediction signal $S_D$ generated using the present invention may be used to improve the accuracy of the anatomical model. In particular, contact between the catheter and the anatomical structure may result in the catheter deforming the anatomical structure. This would lead to the imaging system inaccurately determining a shape of the anatomical structure (e.g. compared to a non-deformed anatomical structure).

The prediction signal may therefore be used by the imaging system to disregard data (for generating the anatomical model) when the catheter is predicted to be touching the anatomical model (with more than a threshold amount of force), e.g. when too high a force is applied by the catheter. This increases an accuracy of the anatomical model, and therefore of a clinicians understanding of the state of the anatomical structure.

In some examples, a visual representation of the anatomical model may be modified to indicate a location of a touch, e.g. by tracking the position of the catheter and detecting when a touch occurs.

Information on whether or not the catheter is touching the anatomical structure can be used to improve the generation of the anatomical model of the anatomical structure. For example, positions of the catheter that represent positions of contact with the anatomical structure (e.g. obtained using the proposed method) could be weighted more than positions of the catheter that do not represent positions of contact with the anatomical structure. This approach can improve the generation of the anatomical model, as positions of the catheter when it (just) makes contact with the anatomical structure mark the true boundaries of the anatomical structure. This concept for improving the generation of an anatomical model using contact information could form a separate inventive concept.

Thus, an aspect of the present disclosure provides a computer-implemented method for generating an anatomical model of an anatomical structure, the method comprising steps of: obtaining position information that identifies a plurality of relative positions of a catheter in a three-dimensional space; obtaining contact information that identifies, for each relative position, whether or not contact is made between the catheter and the anatomical structure and generating an anatomical model of the anatomical structure using the position information and the contact information.

The contact information could be generated using methods described elsewhere in this application, e.g. using the first and second algorithmic process approach.

An appropriate processor circuit and/or computer program product can be provided that performs this computer-implemented method.

Just as contact information (e.g. the predictive signal) can be used to improve the accuracy of a mapping, some embodiments may use mapping to improve contact detection.

The ablation system may comprise an ablation controller 110, previously described. The ablation controller 110 is configured to control the ablation performed by ablation electrodes of the catheter 100.

The ablation controller may be configured to (automatically) control the ablation responsive to an indication of whether or not contact is made between the catheter 100 and the anatomical structure (from the predictive signal $S_P$). In particular, the ablation controller may be configured to control an ablation current provided to the ablation electrodes of the catheter 100 responsive to the predictive signal $S_P$.

For example, the ablation controller may be configured to provide ablation only when contact is made between the catheter and the anatomical structure (and is in the correct position). As another example, the ablation controller may be configured to control a magnitude of ablation responsive to a contact force between the catheter and the anatomical structure.

It is recognized that, in order to ablate efficiently, the catheter should not touch the tissue either too lightly or firmly. For example, if the catheter touches the tissue too lightly, then ablation will be efficient, whereas if the catheter touches the tissue too firmly it can create a hole during the ablation.

Other examples of (automatically) controlling the ablation based on a prediction of whether or not contact is made between the catheter and the anatomical structure will be apparent to the skilled person.

The ablation system 10 may comprise a display or user interface 510.

The processor circuit may be configured to generate a display signal $S_D$ responsive to the prediction of whether or not the catheter is in contact with the anatomical structure (e.g. the predicted contact force).

The display 510 may be configured to receive the display signal $S_D$ and control a visual output of the display based on the display signal. In this way, a clinician or operator can be provided with an indication of whether or not contact has been made and optionally a level of the predicted contact force.

The display signal may, for example, directly control a visual output of the display (e.g. carry display data) of the prediction of whether or not the catheter is in contact with the anatomical structure (and optionally a predicted contact force), or indirectly control (e.g. by carrying data which is subsequently interpreted by the display or user interface).

The display signal may comprise any suitable type of communication, such as an electrical signal (e.g., digital electrical signal) representative of image data or other data for influencing the display. For example, the electrical signal may be in a format suitable for display by a display (such as a computer monitor, television screen, mobile computing device display, etc.).

In some examples, the display 510 may be adapted to provide the visual representation of the anatomical model generated by the imaging system 505 (if present). The visual representation may also display a relative location of the catheter with respect to the anatomical model. The display 510 may be adapted to modify the visual representation of the anatomical model based on the display signal $S_D$, e.g. to indicate a location of a touch.

The ablation system 10 may comprise a user-perceptible alerting device 520 (e.g. an alarm or buzzer). The user-perceptible alerting device may be configured to provide a user-perceptible output, e.g. an audio, visual or haptic output.

The processor circuit may be configured to generate an alert signal $S_A$ that controls the user-perceptible output provided by user-perceptible alerting device responsive to the prediction of whether or not the catheter is in contact with the anatomical structure. One or more characteristics of the user-perceptible output may be adjusted responsive to a detected contact force.

For example, where the user-perceptible alerting device is adapted to provide an audio output, the processor circuit may be configured to cause the alerting device to generate a tone in response to detecting that the catheter is in contact with the anatomical structure (and no tone if no contact is made). In some examples, one or more characteristics of this tone (e.g. volume, frequency, pattern etc.) changes as a contact force increases (e.g. increases in pitch).

This signal may, for example, be provided to a display or user interface that generates a display responsive to the generated signal. In some examples, the signal is provided to a storage, for storing generated indicators (e.g. for later processing or review). In yet other examples, the signal is provided to an alarm module that, responsive to the signal meeting some predetermined criteria, generates a user-perceptible alarm. Other uses and purposes for the signal will be apparent to the skilled person.

The signal may, for example, directly control a visual output of the display (e.g. carry display data) of the two or more indicators and/or additional information derived therefrom, or indirectly control (e.g. by carrying data which is subsequently interpreted by the display or user interface).

In some examples, the signal may comprise any suitable type of communication, such as an electrical signal (e.g., digital electrical signal) representative of image data or other data for influencing the display. For example, the electrical signal may be in a format suitable for display by a display device (e.g., computer monitor, television screen, mobile computing device display, etc.).

The electrodes used in the present disclosure may be repurposed electrodes already existing on/in the ablation catheter, such as ablation electrodes or electrodes used to map the anatomical structure and/or locate the ablation catheter. Thus, the present disclosure has a further advantage in not requiring additional electrodes to be mounted on the ablation catheter.

Previously described embodiments have been provided in the context of measuring a voltage response at the electrode(s) and (for a first algorithmic process) determining an impedance between two electrodes. However, the skilled person would be readily capable of adapting the described approaches for other electrical responses/measurements associated an electrode to a change in an electric field. For example, the impedance-based approach suggested by WO 2019/215721 could be modified to use a different impedance processing algorithm responsive to whether or not ablation is currently being performed.

A combination of different types of response (e.g. voltage response, impedance responses) may be used by the algorithmic processes. In some examples, the first algorithmic process uses (only) a first type of response, with the second algorithmic process using (only) a second type of response. This can allow a more accurate contact-detection approach to be used when no ablation is performed, but a more reliable contact-detection approach (e.g. one less affected by ablation) to be performed when ablation is being carried out.

Fig. 6 is a schematic diagram of a processor circuit 150, according to embodiments of the present disclosure. As shown, the processor circuit 150 may include a data processor 160, a memory 164, and a communication module 168. These elements may be in direct or indirect communication with each other, for example via one or more buses.

The processor circuit 150 may be formed as a single device or may be distributed across a plurality of devices (e.g. a cloud-computing network).

The data processor 160 may include a central processing unit (CPU), a digital signal processor (DSP), an ASIC, a controller, an FPGA, another hardware device, a firmware device, or any combination thereof configured to perform the operations described herein. The data processor160 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 164 may include a cache memory (e.g., a cache memory of the data processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 164 includes a non-transitory computer-readable medium. The non-transitory computer-readable medium may store instructions. For example, the memory 164, or non-transitory computer-readable medium may have program code recorded thereon, the program code including instructions for causing the processor circuit 150, or one or more components of the processor circuit 150, to perform the operations described herein. For example, the processor circuit 150 can execute operations of the methods 200, 500, 700. Instructions 166 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 164, with the code recorded thereon, may be referred to as a computer program product.

The communication module 168 can include any electronic circuitry and/or logic circuitry to facilitate direct or indirect communication of data between the processor circuit 150, the mapping and guidance system 114, the catheter 100, the cryoballoon catheter 130, and/or the display 510. In that regard, the communication module 168 can be an input/output (I/O) device. In some instances, the communication module 168 facilitates direct or indirect communication between various elements of the processor circuit 150 and/or the system 10 (Fig. 1).

As previously explained, the processor circuit 150 may further comprise an electrical signal generator and/or an electrical signal measurer configured to control the electrodes of the catheter to emit and/or detect electrical signals, including voltages, impedances, and currents.

Methods of tracking the position of a catheter and generating an anatomical model of an anatomical structure from the positions of the catheter have been described in this disclosure. Once an anatomical model of an anatomical structure is available, subsequent positions of the catheter with respect to the anatomical model/structure can be tracked, e.g. by monitoring the position of the catheter with respect to the same 3D space from which the anatomical model was generated.

This information can be processed to predict a distance (a "model-derived distance") between the catheter and the anatomical structure. This distance is sometimes called the distance-to-mesh (i.e. the distance between the catheter and the anatomical model - which is usually a mesh). It will be apparent that the model-derived distance will change as the catheter is moved with respect to the anatomical structure.

The model-derived distance can be used to predict whether or not contact is made between the catheter and the anatomical structure. For example, where the model-derived distance is 0, the model predicts that the catheter is in contact with the anatomical structure. Negative values of model-derived distances may represent a magnitude of contact force between the catheter and the anatomical structure, whereas positive values may represent a gap between the catheter and the anatomical structure. This model-derived prediction of contact is largely independent of the occurrence of ablation.

In particular examples, touch information responsive to a touch between the catheter and the anatomical structure can be used to calibrate a model-derived distance. The predictive signal proposed by previously described embodiments carries touch information according to one example of this concept. In particular, the touch information may be generated independently of the anatomical model (e.g. by directly processing electrical response(s) of electrode(s) of a catheter without generating an anatomical model).

Once the touch information indicates a contact is made between the catheter and the anatomical structure (e.g. using herein described algorithmic processes that use catheter electrodes), the model-derived distance at this location can be recorded. In particular, the model-derived distance can be recorded the first time that the touch information indicates that the catheter makes contact with the anatomical structure. This recorded model-derived distance provides a calibration or reference distance.

The difference between this "recorded model-derived distance" and a subsequently determined model-derived distance (e.g. an "ongoing model-derived distance") can more accurately represent a magnitude of touch or contact force (or distance) between the catheter and the anatomical structure.

In particular, if there is no change in the ongoing model-derived distance from the recorded model-derived distance, then the catheter makes a same level of contact with the anatomical structure. If the recorded distance is greater than the ongoing distance, then the catheter is applying a greater contact force to the anatomical structure. If the recorded distance is less than the ongoing distance, then the catheter is moving away from the anatomical structure (i.e. a gap is forming).

Thus, a difference between the recorded distance and the ongoing distance can indicate a magnitude of contact force and/or a distance between the catheter and the anatomical structure.

The difference between the recorded distance and the ongoing distance may be compared to one or more thresholds to identify one or more predicted touch levels (e.g. no touch, high touch, very high touch) between the catheter and the anatomical structure. Thus, additional contact information and/or context can be derived from the difference.

It is also recognized that if the recorded model-derived distance is too large or too small (e.g. too far below zero), it can be predicted that the mapping or creation of the anatomical model was inaccurate. Thus, some embodiments may comprise a step of comparing the recorded model-derived distance to one or more predetermined thresholds and, if the recorded model-derived distance breaches the threshold(s), generating a model accuracy alert. The model accuracy alert may cause a model generation (for generating the anatomical model) to be repeated.

An aspect of the present disclose provides a computer-implemented method for generating a measure of distance or contact force between a catheter, comprising one or more electrodes, and an anatomical structure.

There is proposed a computer-implemented method of:

obtaining an anatomical model of the anatomical structure;
monitoring a position of the catheter with respect to the anatomical model;
monitoring a model-derived distance between the catheter and the anatomical structure by processing the anatomical model and the determined position of the catheter with respect to the anatomical model;
obtaining touch information indicating a prediction of whether or not the catheter is in contact with the anatomical structure;
in response to the touch information indicating that the catheter makes contact with the anatomical structure, recording the model-derived distance; and
determining a difference between the recorded model-derived distance and an ongoing model-derived distance to thereby generate a measure of distance or contact force between the catheter and the anatomical structure.

Preferably, the touch information is independent of the anatomical model, i.e. is not generated using an anatomical model of the anatomical structure. For example, the touch information may be generated by processing an impedance between two electrodes of the catheter or other electrical response responsive to a contact between the catheter and the anatomical structure.

The method may comprise further generating information responsive to the measure of distance or contact force between the catheter and the anatomical structure. This process may comprise, for example, categorizing the touch level or distance level by comparing the measure of distance or contact force to one or more thresholds.

The measure of distance or contact force or information derived therefrom may be presented to a user, e.g. at a user interface, to improve their understanding of the relationship between the catheter and the anatomical structure.

The proposed approach provides a method of generating a model-derived measurement of distance and/or contact force between the catheter and the anatomical structure which is calibrated. This facilitates a mechanism for deriving such a measurement.

The proposed approach recognizes that, mapping (i.e. generation of the anatomical model) may be incomplete and/or inaccurate, such that a calibrated model-derived measurement may also be inaccurate. Rather, the proposed approach provides a mechanism by which such inaccuracies can be overcome through use of calibration using a predictive signal.

Similarly, the proposed approach recognizes that the determined position of the catheter (used to generate the model-derived distance) may not coincide with the actual point of touch with the anatomical structure, and the relationship between determined position and point of touch may be previously unknown.

The touch information may, for example, be contained in a predictive signal generated using any previously described method for obtaining a predictive signal.

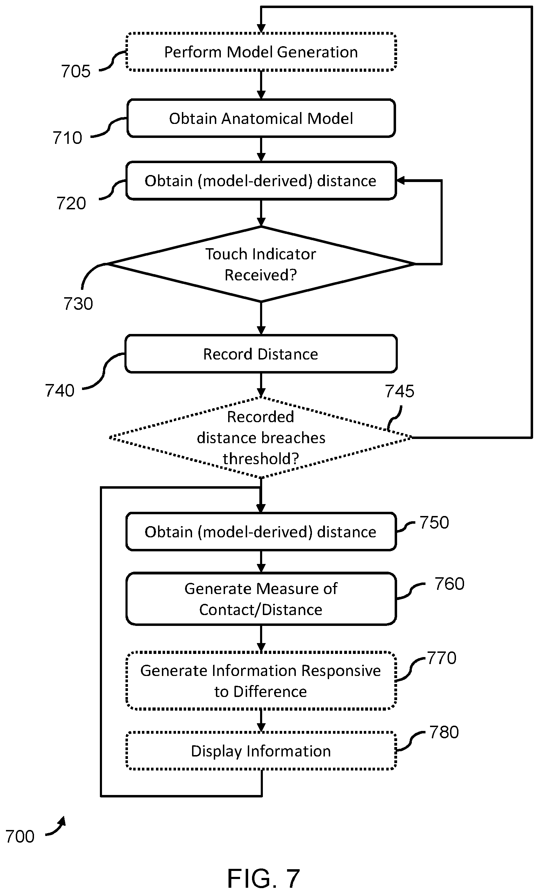

Fig. 7 illustrates a method according to this aspect of the present disclosure. In particular, Fig. 7 illustrates a method 700 for generating a measure of distance or contact force between a catheter and the anatomical structure.

The method 700 may comprise a step 705 of generating an anatomical model of the anatomical structure. This may be performed using positions of the catheter (or of another, different catheter). Methods of generating an anatomical model have been previously identified.

The method 700 comprises a step 710 of obtaining the anatomical model. The anatomical model may, for example, be obtained from an anatomical model generator and/or a memory.

The method 700 further comprises a step 720 of obtaining a model-derived distance between the catheter and the anatomical structure. This can be performed by determining a relative position of the catheter with respect to the anatomical model, and deriving the model-derived distance based on this position and the anatomical model.

The method 700 further comprises a step 730 of determining whether or not an indication of a touch between the catheter and the anatomical structure has been received. This indication may be contained in touch information generated by monitoring one or more electrodes of the catheter. Preferably, this touch information is not generated using the anatomical model.

The method 700 further comprises a step 740 of, in response to the touch information indicating a touch between the catheter and the anatomical structure, recording (i.e. committing to memory) the model-derived distance.

The method 700 may further comprise a step 745 of comparing the recorded model-derived distance to a threshold. If the model-derived distance breaches a threshold (e.g. is too small or too large), the method may revert back to step 705. In particular, if the model-derived distance is too large or too small, this may indicate that the model is insufficiently accurate for providing clinically acceptable clinical guidance.

The method 700 comprises a step 750 of obtaining the model-derived distance between the catheter and the anatomical structure. This can be performed using the same process as step 720.

The method 700 also comprises a step 760 of generating a measure of contact force and/or distance between the catheter and the anatomical structure using the recorded model-derived distance (obtained in step 740) and the model-derived distance obtained in step 750. In particular, a difference between these two distances may represent a (calibrated) distance between the catheter and the anatomical structure and/or a measure of contact force.

The method 700 may comprise a step 770 of generating information responsive to the difference, e.g. by comparing the difference to one or more predetermined thresholds to categories the difference.

The method 700 may comprise a step 780 of displaying information responsive to the difference, e.g. at a user interface. This information may comprise the difference itself and/or data (such as the categories described with reference to step 770) derived from the difference.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The computer-readable program may execute entirely on a single computer/processor, partly on the computer/processor, as a stand-alone software package, partly on the computer/processor and partly on a remote computer or entirely on the remote computer or server (e.g. using a distributed processor processing system). In the latter scenario, the remote computer may be connected to the computer/processor through any type of network, including a local area network (LAN) or a wide area network (WAN), or the connection may be made to an external computer (for example, through the Internet using an Internet Service Provider).

It will also be understood that the embodiments described above are exemplary and are not intended to limit the scope of the disclosure to a given clinical application. For example the devices, systems, and techniques described above can be used in a variety of ablation applications that involve the ablation of feature of an anatomical structure. The techniques described above can be used to guide an RF ablation procedure in which one or more RF ablation electrodes are used to create an electrically-isolating lesion in cardiac tissue. Of course, the techniques described above could also be used to guide any other suitable ablation procedure, such as a cryo-ablation or pulsed field ablation procedure.

Further, while the ablation procedures are described with respect to the heart and associated anatomy, it will be understood that the same methods and systems can be used for ablation procedures in other body volumes, including other regions of interest in the heart, or other body cavities and/or lumens. For example, in some embodiments, the procedures described herein can be used alongside treatment procedures in any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. The anatomy may be a blood vessel, as an artery or a vein of a patient's vascular system, including cardiac vasculature, peripheral vasculature, neural vasculature, renal vasculature, and/or any other suitable lumen inside the body. In addition to natural structures, the approaches described herein may be used to examine man-made structures such as, but without limitation, heart valves, stents, shunts, filters and other devices in the kidneys, lungs, or any other suitable body volume. Thus, the anatomical structure referred to in the present disclosure may include any suitable organ (such as those previously listed), blood vessel and/or man-made structures.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

**Claims**

1. A processor circuit (150) for generating a predictive signal ($S_P$) indicative of if an ablation therapy catheter (100) is in contact with an anatomical structure (190), the processor circuit being configured for communication with an ablation therapy catheter comprising two or more electrodes (101, 102, 103, 104), the processor circuit comprising:

   an input interface (151) configured to obtain (210, 220):

   from the two or more electrodes of the ablation catheter, at least one electrical response ($V_{1,1}$, $Z_{1,2}$, $V_{2,3}$, $Z_{3,4}$) of each electrode of the ablation therapy catheter to one or more electric fields in the anatomical structure; and
   from the electrodes of the ablation therapy catheter or a controller of the ablation therapy catheter, an indicator signal responsive to an indication of whether or not the ablation therapy catheter is ablating the anatomical structure, and

   a data processor (160) communicatively coupled to the input interface and configured to:

   in response to the indicator signal indicating that the ablation therapy catheter is not ablating the anatomical structure, use a first algorithmic process (230) to process a first set of one or more electrical responses of one or more electrodes of the ablation therapy catheter to predict whether or not the ablation therapy catheter is in contact with the anatomical structure; and
   in response to the indicator signal indicating that the ablation therapy catheter is ablating the anatomical structure, use a second, different algorithmic process (240) to process a second set of one or more electrical responses of one or more electrodes of the ablation therapy catheter to predict whether or not the ablation therapy catheter is in contact with the anatomical structure; and
   output (250) a predictive signal responsive to the prediction of whether or not the ablation therapy catheter is in contact with the anatomical structure.

2. The processor circuit (150) of claim 1, wherein the two or more electrodes of the ablation therapy catheter (100) comprise one or more electrodes (101) used to apply an ablation treatment to the anatomical structure.

3. The processor circuit (150) of any of claims 1 or 2, wherein:

   the first set of one or more electrical responses comprises one or more electrical responses ($Z_{1,2}$) of a first set of one or more electrodes (101, 102) of the ablation therapy catheter; and
   the second set of one or more electrical responses comprises one or more electrical responses ($V_{2,3}$) of a second, different set of one or more electrodes (102, 103) of the ablation therapy catheter.

4. The processor circuit (150) of claim 3, wherein the first set of one or more electrodes comprises a most distally positioned electrode (101) of the ablation therapy catheter.

5. The processor circuit (150) of claim 4, wherein the second set of one or more electrodes does not comprise the most distally positioned electrode (101) of the ablation therapy catheter.

6. The processor circuit (150) of any of claims 3 to 5, when dependent upon claim 2, wherein the first set of electrodes comprises at least one of the one or more electrodes (101) used to apply an ablation treatment to the anatomical structure.

7. The processor circuit (150) of claim 6, wherein the second set of electrodes does not comprise the at least one of the one or more electrodes (101) used to apply an ablation treatment to the anatomical structure.

8. The processor circuit (150) of any of claims 3 to 7, when dependent upon claim 2, wherein the second set of electrodes does not comprise any of the electrodes (101) used to apply an ablation treatment to the anatomical structure.

9. The processor circuit (150) of any of claims 1 to 8, wherein the first algorithmic process comprises determining an impedance measure ($Z_{1,2}$), of an impedance between two electrodes (101, 102), using the electrical responses of the two electrodes to an electric field generated at one of the two electrodes.

10. The processor circuit (150) of claim 9, wherein at least one of the two electrodes (101) associated with the impedance measure is an ablation electrode used to apply an ablation treatment to the anatomical structure.

11. The processor circuit (150) of claim 10, wherein at least one of the two electrodes associated with the impedance measure is a most distally positioned electrode (101) of the ablation therapy catheter.

12. The processor circuit (150) of any of claims 9 to 11, wherein the second algorithmic process does not comprises determining an impedance measure of an impedance between two electrodes.

13. The processor circuit (150) of any of claims 1 to 12, further comprising an output interface (152) configured to provide the predictive signal ($S_P$) responsive to the prediction of whether or not the ablation therapy catheter is in contact with the anatomical structure.

14. The processor circuit (150) of any of claims 1 to 13, wherein the processor circuit further comprises an electrical signal generator configured to control a current supplied to each of the two or more electrodes to thereby control an electric field generated by each electrode.

15. A method (200) for predicting if an ablation therapy catheter (100), comprising two or more electrodes (101, 102, 103, 104), is in contact with an anatomical structure (190), the method comprising:

obtaining (210) at least one electrical response ($V_{1,1}$, $Z_{1,2}$, $V_{2,3}$, $Z_{3,4}$) of each electrode of the ablation therapy catheter to one or more electric fields in the anatomical structure;
obtaining (220) from the electrodes of the ablation therapy catheter or a controller of the ablation therapy catheter, an indicator signal responsive to an indication of whether or not the ablation therapy catheter is ablating the anatomical structure,
in response to the indicator signal indicating that the ablation therapy catheter is not ablating the anatomical structure, using (230) a first algorithmic process to process a first set of one or more electrical responses of one or more electrodes of the ablation therapy catheter to predict whether or not the ablation therapy catheter is in contact with the anatomical structure; and
in response to the indicator signal indicating that the ablation therapy catheter is ablating the anatomical structure, using (240) a second, different algorithmic process to process a second set of one or more electrical responses of one or more electrodes of the ablation therapy catheter to predict whether or not the ablation therapy catheter is in contact with the anatomical structure; and
outputting (250) a predictive signal responsive to the prediction of whether or not the ablation therapy catheter is in contact with the anatomical structure.

16. The method of claim 15, wherein the two or more electrodes of the ablation therapy catheter comprise one or more electrodes used to apply an ablation treatment to the anatomical structure.

17. The method of any of claims 15 or 16, wherein:

the first set of one or more electrical responses comprises one or more electrical responses of a first set of one or more electrodes of the ablation therapy catheter; and

the second set of one or more electrical responses comprises one or more electrical responses of a second, different set of one or more electrodes of the ablation therapy catheter.

**18.** A computer program product comprising code which, when executed by a processor circuit, causes the processor circuit to perform the steps of the method according to any one of steps 15 to 17.

**19.** A non-transitory computer-readable medium or data carrier comprising or carrying the computer program product of claim 18.

110

195

190

100 106

101 102 103 104

151

150

160

151

10

152

S_P

## FIG. 1

210 Obtain Electrical Responses

220

N

Ablation?

Y

230 Use 1st Algorithmic Process

240 Use 2nd Algorithmic Process

250 Output Predictive Signal

200

## FIG. 2

310

$Z_{1,2}$

320

$V_{2,3}$

$T_1$    $T_2$   $T_3$     $T_4$     $T_5$    $T_6$

FIG. 3

$V_{1,1}$     $Z_{1,2}$       103     104

101

102    $V_{2,3}$    $Z_{3,4}$

FIG. 4

FIG. 5

FIG. 6

FIG. 7

Europäisches
Patentamt

European
Patent Office

Office européen
des brevets

## EUROPEAN SEARCH REPORT

Application Number

EP 20 18 3219

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/067628 A1 (ST JUDE MEDICAL ATRIAL FIBRILL [US]; PAUL SAURAV [US] ET AL.) 14 June 2007 (2007-06-14) * paragraph [0071]; figure 8 * ----- | 1,2,13, 14 | INV. A61B18/14 A61B90/00 A61B18/00 |
| X | US 2019/038348 A1 (KOBLISH JOSEF VINCENT [US] ET AL) 7 February 2019 (2019-02-07) * figures 26c,27,42a,42b * * paragraph [0570] * * paragraph [0570] * * paragraph [0572] * * paragraph [0573] * * paragraph [0692] - paragraph [0693] * ----- | 1,2 | |
| X,D | WO 2019/215721 A1 (NAVIX INTERNATIONAL LTD) 14 November 2019 (2019-11-14) * page 25, paragraph 26 - page 26, line 6 * ----- | 1-11,14 | |
| X | US 2014/243813 A1 (PAUL SAURAV [US] ET AL) 28 August 2014 (2014-08-28) * paragraphs [0144] - [0145] * * paragraph [0024] * * paragraph [0145] * ----- | 1-8 | TECHNICAL FIELDS SEARCHED (IPC) A61B |

The present search report has been drawn up for all claims

| Place of search | Date of completion of the search | Examiner |
|---|---|---|
| The Hague | 10 December 2020 | Cornelissen, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 18 3219

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2020

| Patent document cited in search report | Publication date | Patent family member(s) | | Publication date |
|---|---|---|---|---|
| WO 2007067628 A1 | 14-06-2007 | AU | 2006321570 A1 | 14-06-2007 |
| | | AU | 2006321574 A1 | 14-06-2007 |
| | | AU | 2006321918 A1 | 14-06-2007 |
| | | BR | PI0619725 A2 | 11-10-2011 |
| | | BR | PI0621017 A2 | 29-11-2011 |
| | | BR | PI0621018 A2 | 29-11-2011 |
| | | CA | 2631940 A1 | 14-06-2007 |
| | | CA | 2631946 A1 | 14-06-2007 |
| | | CA | 2632604 A1 | 14-06-2007 |
| | | CN | 101534736 A | 16-09-2009 |
| | | CN | 103251451 A | 21-08-2013 |
| | | EP | 1962708 A2 | 03-09-2008 |
| | | EP | 1962710 A2 | 03-09-2008 |
| | | EP | 1962945 A1 | 03-09-2008 |
| | | IL | 191729 A | 30-06-2015 |
| | | IL | 191730 A | 30-04-2012 |
| | | IL | 191733 A | 30-06-2014 |
| | | JP | 5162467 B2 | 13-03-2013 |
| | | JP | 5312948 B2 | 09-10-2013 |
| | | JP | 5426171 B2 | 26-02-2014 |
| | | JP | 2009518130 A | 07-05-2009 |
| | | JP | 2009518150 A | 07-05-2009 |
| | | JP | 2009518151 A | 07-05-2009 |
| | | US | 2008275465 A1 | 06-11-2008 |
| | | US | 2008281319 A1 | 13-11-2008 |
| | | US | 2008288038 A1 | 20-11-2008 |
| | | US | 2008300589 A1 | 04-12-2008 |
| | | US | 2010228247 A1 | 09-09-2010 |
| | | US | 2010241117 A1 | 23-09-2010 |
| | | US | 2013138099 A1 | 30-05-2013 |
| | | US | 2014364843 A1 | 11-12-2014 |
| | | US | 2018078300 A1 | 22-03-2018 |
| | | WO | 2007067628 A1 | 14-06-2007 |
| | | WO | 2007067937 A2 | 14-06-2007 |
| | | WO | 2007067938 A2 | 14-06-2007 |
| | | WO | 2007067939 A2 | 14-06-2007 |
| | | WO | 2007067941 A2 | 14-06-2007 |
| | | WO | 2007067943 A2 | 14-06-2007 |
| US 2019038348 A1 | 07-02-2019 | CN | 110809448 A | 18-02-2020 |
| | | EP | 3614946 A1 | 04-03-2020 |
| | | US | 2019038348 A1 | 07-02-2019 |
| | | US | 2019038349 A1 | 07-02-2019 |
| | | WO | 2018200865 A1 | 01-11-2018 |
| WO 2019215721 A1 | 14-11-2019 | NONE | | |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 20 18 3219

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2020

| Patent document cited in search report | Publication date | Patent family member(s) | Publication date |
|---|---|---|---|
| US 2014243813 A1 | 28-08-2014 | EP 2197377 A1 <br> US 2010274239 A1 <br> US 2014243813 A1 <br> US 2017312009 A1 <br> WO 2009065140 A1 | 23-06-2010 <br> 28-10-2010 <br> 28-08-2014 <br> 02-11-2017 <br> 22-05-2009 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019215721 A1
- US 2012283715 A1
- US 2018310987 A
- US 10278616 B
- US 5983126 A
- US 6002955 A
- WO 2019215721 A

**Non-patent literature cited in the description**

- **ROMANOV, ALEXANDER et al.** High-resolution, real-time, and nonfluoroscopic 3-dimensional cardiac imaging and catheter navigation in humans using a novel dielectric-based system. *Heart rhythm,* 2019, vol. 16.12, 1883-1889